# DEMANDE DE BREVET EUROPEEN

(11) **EP 1 884 248 A1**
(43) Date de publication de la demande: **06.02.2008**
(21) Numéro de dépôt: 06300847.8
(22) Date de dépôt: 01.08.2006
(51) Int. Cl.: A61L 2/14, B08B 7/00

(54) **Procédé continu de fonctionnalisation et aseptisation d'éléments plans d'emballages alimentaires par plasma atmosphérique**

(71) Demandeur: L'AIR LIQUIDE, Société Anonyme pour l'Etude et l'Exploitation des Procédés Georges Claude, 75007 Paris (FR)
(72) Inventeur: ROSTAING, Jean-Christophe, 78000 Versailles (FR); COCOLIOS, Panayotis, 78830 Bullion (FR)
(74) Mandataire: Mellul-Bendelac, Sylvie Lisette

(57) **Abrégé**

L'invention porte sur un procédé de fonctionnalisation et stérilisation d'éléments plans d'emballages alimentaires, ledit procédé étant conduit à pression atmosphérique et en continu à l'aide d'une source plasma de décharge atmosphérique froide à barrière diélectrique et/ou une source de plasma micro-ondes atmosphérique plane.

## Description

Des traitements par plasmas froids atmosphériques (corona ou à barrière diélectrique (DBD)) sont utilisés pour fonctionnaliser les surfaces de films de polymères mis en oeuvre pour l'emballage alimentaire. Ces traitements ont notamment pour but d'améliorer l'aptitude des films de polymères à être collés, imprimés, etc... L'utilisation d'atmosphères contrôlées plutôt que d'air comme gaz où est entretenu le plasma permet d'améliorer les performances du procédé. En particulier de tels traitements, tels que ceux rapportés dans le document WO 01/58992 permettent d'atteindre des valeurs particulièrement élevées de l'énergie de surface, et en outre cette activation du film traité persiste pour un temps relativement long. Cet avantage permet un stockage prolongé des rouleaux de films avant les opérations de traitement ultérieures, et apporte ainsi une plus grande souplesse dans la gestion de la chaîne de transformation.

De nombreuses études fondamentales sur les propriétés de la décharge et sur les processus microscopiques de modification physicochimiques des surfaces des polymères ont permis d'optimiser la composition du gaz et l'ensemble des autres paramètres du procédé. Par exemple, il a été mis en évidence que lorsqu'un gaz oxydant est requis, il est préférable d'utiliser du protoxyde d'azote N₂O que de l'oxygène O₂. L'emploi de N₂O confère une meilleure stabilité et un meilleur contrôle de la décharge, et réduit les dommages potentiels au film dus à l'attaque par des radicaux oxydants agressifs.

Aujourd'hui, outre les propriétés fonctionnelles des films, on cherche à avoir des films aseptisés ou stérilisés avant utilisation. L'aseptisation en ligne est connue depuis longtemps dans le cas des matériaux minces multicouches (carton/aluminium/polymère) mis en oeuvre dans le conditionnement des aliments liquides où les « briques » sont formées, remplies et scellées en une seule opération continue. Dans ce cas l'agent aseptisant est un désinfectant chimique comme le peroxyde d'hydrogène ou l'acide peracétique. Ces produits sont des polluants et produisent des effluents dont les coûts de traitements s'ajoutent au prix de revient de l'opération de transformation.

Il est connu que les plasmas atmosphériques froids peuvent également permettre de désinfecter et stériliser des surfaces solides. C'est l'état de plasma qui est spécifiquement à l'origine des propriétés germicides des gaz et on peut utiliser des gaz de procédé simples tels que par exemple, l'azote, les gaz rares, l'oxygène et/ou le protoxyde d'azote, qui ne sont pas polluants.

Jusqu'ici ce type de technologie est développé principalement pour des applications de stérilisation biomédicale, que ce soit à basse pression ou à pression atmosphérique. Récemment, la littérature (par exemple par exemple l'équipe Benhacene-Boudam et al. de l'Université de Montréal et l'Université Paul Sabatier de Toulouse/France), ont étudié les mécanismes de stérilisation par un plasma de décharge atmosphérique luminescente dans l'azote avec un très faible pourcentage de N₂O. Il a été montré que les photons ultra-violets émis par la désexcitation du niveau y de NO (ce dernier produit à faible concentration par réaction chimique dans la décharge) jouent un rôle majeur dans les mécanismes d'inactivation des germes. Plus précisément le maximum d'efficacité de stérilisation est obtenue pour les conditions opératoires qui maximisent l'émission de ces photons UV. Ce résultat est similaire à celui observé généralement à basse pression dans des post-décharges N₂/O₂, Ar. Il est décrit dans les demandes de brevet WO 2000/072889 et WO 2004/011039 au nom de l'Université de Montréal. Ceci vaut du moins pour des conditions plasma « douces » nécessaires pour ne pas endommager des dispositifs biomédicaux coûteux réutilisables un grand nombre de fois.

Les traitements envisagés durent alors quelques minutes.

Dans ces applications de stérilisation de matériel biomédical spécifiques, la durée de la stérilisation n'est pas un obstacle.

Dans l'industrie de l'emballage alimentaire, il est au contraire essentiel d'avoir des temps de traitement très courts de façon à ne pas ralentir les chaînes de fabrication sur lesquelles la vitesse de convoyage des films est de l'ordre de plusieurs centaines de mètres par minutes.

Ainsi, il existe un besoin réel en un procédé permettant la fonctionnalisation et la stérilisation d'éléments plans d'emballage qui soit non polluant et très rapide, de façon à pouvoir être intégré dans des lignes d'emballage classiques.

Les inventeurs ont trouvé qu'un procédé de fonctionnalisation et stérilisation d'éléments plans d'emballages alimentaires, conduit à pression atmosphérique et en continu, à l'aide d'une source plasma de décharge atmosphérique froide à barrière diélectrique et/ou une source de plasma micro-ondes atmosphérique plane satisfaisait ce besoin.

Dans la présente demande, par «stérilisation » on entend aussi bien aseptisation que stérilisation. Les deux termes sont utilisés indifféremment. En effet, dans le domaine des emballages alimentaires, l'obtention d'une stérilité totale à 0 germe n'est pas nécessaire car le produit alimentaire à emballer n'est pas lui-même stérile. En fait, ce que l'on recherche ici, c'est que le film d'emballage n'augmente pas le niveau résiduel initial de concentration bactérienne dans le produit et donc ne réduise pas le délai au bout duquel les bactéries se multipliant vont atteindre un niveau rendant le produit impropre à la consommation.

On entend par « fonctionnalisation » un traitement de surface destiné à introduire à la surface du substrat à traiter des fonctionnalités/groupements chimiques tels que des groupements amine, amide, nitrile, ou encore carbonyle, carboxyle et autre alcool ou ester, et changer ainsi les propriétés de surface du substrat à traiter, par exemple pour lui conférer une certaine hydrophilie, ou une meilleure adhésivité.

L'invention est destinée à des éléments d'emballages plans, c'est-à-dire à des substrats choisis dans le groupe comprenant les substrats se présentant sous forme de feuilles, films, fibres tissées ou non tissées et/ou mousses. Le substrat est en céramique, verre, métal, cartons, matériaux composites ou polymères. De préférence le substrat est un polymère choisi dans le groupe comprenant une polyoléfine, un polymère vinylique, un polystyrène, un polyester, un polyamide ou un polycarbonate.

Dans la présente invention, on utilisera le terme « film » pour désigner indifféremment chacun de ces types de substrats.

Ces films sont ensuite stockés en rouleaux avant d'être mis en forme pour réaliser des emballages, notamment de type briques.

Lorsque ces films sont destinés à être stockés en rouleaux avant transformation en emballages, il est important que le traitement de stérilisation soit réalisé simultanément sur les deux faces du film.

Dans le procédé conforme à l'invention, les étapes de stérilisation et de fonctionnalisation peuvent être réalisées simultanément ou successivement. De préférence elles sont réalisées de façon successive.

L'étape de fonctionnalisation est réalisée en utilisant un plasma tel que décrit notamment dans la demande de brevet WO 01/58992. Le mélange gazeux utile dans le procédé comporte un gaz porteur choisi dans le groupe comprenant l'azote, l'argon, l'hélium ou leurs mélanges, ainsi qu'un gaz réducteur tel que l'hydrogène et/ou un gaz oxydant choisi dans le groupe comprenant CO₂, N₂O, H₂O ou leurs mélanges. Différents types de plasmas peuvent être envisagés pour l'aseptisation. Le plasma standard est du type décharge à barrière diélectrique filamentaire. Un deuxième plasma de ce type peut être utilisé pour l'aseptisation, avec des paramètres opérationnels différents (fréquence et puissance de l'alimentation) et/ou des mélanges gazeux différents.

Il peut être utile alternativement d'utiliser une décharge à barrière diélectrique de type luminescent, selon la demande de brevet WO 00/13202, susceptible de donner une meilleure homogénéité au traitement d'aseptisation.

La décharge à barrière diélectrique peut aussi être alimentée par une source de puissance plus ou moins fortement pulsée. Des impulsions courtes intenses favorisent la génération d'électrons de plus haute énergie, jusqu'à plusieurs dizaines d'électrons-volts et de concentrations élevées de radicaux.

Cependant pour l'application d'aseptisation rapide il peut aussi être indiqué d'utiliser des décharges présentant une densité électronique plus élevée que les DBD et une énergie moyenne des électrons plus basse. C'est notamment le cas des décharges micro-ondes atmosphériques dont la densité électronique dépasse couramment 10¹² cm⁻³ alors que l'énergie électronique moyenne est de l'ordre de 1 à 2 eV.

Sans vouloir être liés par aucune théorie, les présents inventeurs sont d'avis que les mécanismes d'inactivation font intervenir:
- les photons UV émis in-situ par désexcitation d'espèces du plasma, dégradant directement le matériel génétique des micro-organismes ;.
- les radicaux oxydants ou réducteurs susceptibles de diffuser à travers la matière organique et d'atteindre les noyaux des cellules pour avoir une action bactéricide analogue à celle de désinfectants chimiques ;
- les radicaux oxydants et réducteurs pouvant induire une érosion chimique de la matière organique (dégradation de la substance des germes en méthane, anhydride carbonique et eau) ;
- d'autres effets physiques de volatilisation de la matière des germes par désexcitation d'espèces du plasma au contact de la surface.

Ce dernier effet est peu sélectif par rapport à la surface de polymère sous-jacente.

La durée de la stérilisation est de 10⁻³ à 10⁻¹ s, de préférence de 5.10⁻³ à 5.10⁻¹ s, et plus préférentiellement encore de 10⁻³ à 10⁻² s.

Selon un mode de réalisation particulier, les étapes de stérilisation et de fonctionnalisation sont réalisées dans un plasma de décharge à barrière diélectrique filamentaire. Les deux étapes peuvent alors être conduites simultanément, en choisissant un bon compromis pour les paramètres du procédé. Cependant, il est avantageux de modifier les paramètres opérationnels du plasma (fréquence et puissance d'alimentation) et/ou d'utiliser des mélanges gazeux différents pour chacune des étapes. Les étapes sont alors réalisées successivement dans le même appareillage.

Il est également possible de réaliser l'étape de stérilisation dans un plasma de décharge à barrière diélectrique de type luminescent et l'étape de fonctionnalisation dans un plasma de décharge à barrière diélectrique filamentaire. L'utilisation d'un plasma de décharge à barrière diélectrique de type luminescent permet d'obtenir une meilleure homogénéité de la stérilisation.

Une autre possibilité consiste à réaliser l'étape de stérilisation dans un plasma de décharge micro-ondes alors que l'étape de fonctionnalisation est réalisée dans un plasma de décharge à barrière diélectrique filamentaire.

Le plasma de décharge micro-ondes permet l'obtention d'une densité électronique plus élevée que les plasmas de décharge à barrière diélectrique alors que l'énergie moyenne des électrons est plus basse. La densité électronique dépasse couramment 10¹² cm⁻³ et l'énergie électronique moyenne est de l'ordre de 1 à 2 eV avec les décharges micro-ondes atmosphériques classiques.

Selon un premier mode de réalisation, l'étape de fonctionnalisation est conduite avant l'étape de stérilisation.

Les conditions de la stérilisation seront alors choisies de telle sorte que les effets secondaires sur le substrat, par exemple l'ablation de matière, soient aussi limités que possible de façon à ne pas endommager la surface fonctionnalisée.

Selon un second mode de réalisation, l'étape de stérilisation est conduite avant l'étape de fonctionnalisation.

Dans ce cas, les conditions de la stérilisation peuvent être beaucoup plus sévères, que dans le premier mode de réalisation et ainsi la durée de la stérilisation peut encore être raccourcie. Il est alors avantageux de choisir un plasma favorisant les effets physiques de volatilisation de la matière des germes par désexcitation d'espèces du plasma.

Dans ce mode de réalisation, il est possible, après l'étape de fonctionnalisation de conduire une étape supplémentaire de finition de la stérilisation. Cette étape peut notamment être utile si les films d'emballage traités ont été stockés par exemple pendant quelques jours et sont après stockage introduits sur les chaînes d'emballage alimentaire.

Cette étape de finition de stérilisation est extrêmement rapide et conduite dans des conditions douces.

L'invention va être décrite de façon plus détaillée en référence aux exemples suivants qui sont donnés uniquement à titre illustratif.

### EXEMPLE 1

Un film de polypropylène sans additif, d'une épaisseur de 15 microns est convoyé à une vitesse de défilement de 80m/min.

Ce film est traité afin d'en améliorer l'adhésion en le traitant dans un plasma de décharge atmosphérique froide à barrière diélectrique de type filamentaire.

Le mélange gazeux utilisé est un mélange N₂/N₂O/H₂ à 250ppm de N₂O et présentant un rapport H₂/N₂O de 46.

La puissance spécifique de la décharge est de 40 Wmn/m².

A un film identique, on fait subir le même traitement, suivi d'une stérilisation. Pour ceci, le mélange gazeux est alors modifié. Il est enrichi en protoxyde d'azote à hauteur de 1000 ppm et la puissance spécifique de la décharge est conservée. Le film est ainsi stérilisé.

Les énergies de surface de chacun de ces films sont mesurées à la fin du traitement puis après 100 jours. Les valeurs obtenues sont toutes équivalentes.

La stérilisation ne modifie donc pas les caractéristiques obtenues grâce au premier traitement et les propriétés sont stables dans le temps.

### EXEMPLE 2 :

Un film de polyéthylène terephthalate PET de 30 microns d'épaisseur est utilisé comme substrat. Sur celui-ci on a pulvérisé une suspension contenant des spores de *Bacillus Subtilis.* La quantité de spores a été déterminée en pesant une section d'un film témoin et une section équivalente d'un film sur lequel ont été pulvérisés les spores.

Ce substrat est soumis à un premier traitement destiné à améliorer l'adhésion, tel que décrit dans l'exemple 1.

Puis immédiatement après, il est soumis à une de décharge atmosphérique froide à barrière diélectrique de type luminescent en utilisant un mélange gazeux H₂/N₂ avec une puissance spécifique de décharge de 60 Wmin/m². Le traitement total a duré 2 secondes.

A l'issue de ce traitement, la quantité de spores a diminué de 80%.

## Revendications

1. Procédé de fonctionnalisation et stérilisation d'éléments plans d'emballages alimentaires, ledit procédé étant conduit à pression atmosphérique et en continu à l'aide d'une source plasma de décharge atmosphérique froide à barrière diélectrique et/ou une source de plasma micro-ondes atmosphérique plane.

2. Procédé selon la revendication 1, **caractérisé par le fait que** ledit élément plan est choisi dans le groupe comprenant les substrats se présentant sous forme de feuille, film, fibres tissées ou non tissées et/ou mousse.

3. Procédé selon la revendication 1 ou 2, **caractérisé par le fait que** le substrat est en céramique, verre, métal, carton, matériau composite ou polymère.

4. Procédé selon la revendication 3, **caractérisé par le fait que** le polymère est choisi dans le groupe comprenant une polyoléfine, un polymère vinylique, un polystyrène, un polyester, un polyamide ou un polycarbonate.

5. Procédé selon l'une quelconque des revendications 1 à 4, **caractérisé par le fait que** les étapes de fonctionnalisation et de stérilisation sont effectuées successivement.

6. Procédé selon l'une quelconque des revendications 1 à 5, **caractérisé par le fait que** la durée de la stérilisation est de 10⁻³ à 10⁻¹ s, de préférence de 5.10⁻³ à 5.10⁻¹ s, et plus préférentiellement encore de 10⁻³ à 10⁻² s.

7. Procédé selon l'une quelconque des revendications 1 à 6, **caractérisé par le fait que** l'étape de fonctionnalisation est réalisée en utilisant un mélange gazeux comportant un gaz porteur choisi dans le groupe comprenant l'azote, l'argon, l'hélium ou leurs mélanges, ainsi qu'un gaz réducteur tel que l'hydrogène et/ou un gaz oxydant choisi dans le groupe comprenant le CO₂ ou le N₂O ou H₂O ou leurs mélanges.

8. Procédé selon la revendication 1 à 7, **caractérisé par le fait que** les étapes de stérilisation et de fonctionnalisation sont réalisées dans un plasma de décharge à barrière diélectrique filamentaire.

9. Procédé selon la revendication 5, **caractérisé par le fait que** l'étape de stérilisation est réalisée dans un plasma de décharge à barrière diélectrique de type luminescent, l'étape de fonctionnalisation étant réalisée dans un plasma de décharge à barrière diélectrique filamentaire.

10. Procédé selon la revendication 5, **caractérisé par le fait que** l'étape de stérilisation est réalisée dans un plasma de décharge micro-ondes, l'étape de fonctionnalisation étant réalisée dans un plasma de décharge à barrière diélectrique filamentaire.

11. Procédé selon l'une quelconque des revendications 1 à 10, **caractérisé par le fait que** l'étape de fonctionnalisation est conduite avant l'étape de stérilisation.

12. Procédé selon l'une quelconque des revendications 1 à 10, **caractérisé par le fait que** l'étape de stérilisation est réalisée avant l'étape de fonctionnalisation.

13. Procédé selon la revendication 12, **caractérisé par le fait qu'**il comprend, après l'étape de fonctionnalisation, une étape de finition de la stérilisation.

14. Procédé selon l'une quelconque des revendications 1 à 13, **caractérisé par le fait qu'**au moins l'étape de stérilisation est conduite sur chacune des surfaces de l'élément plan d'emballage.
